# EUROPEAN PATENT APPLICATION

(11) **EP 3 967 321 A1**
(43) Date of publication of application: **16.03.2022**
(21) Application number: 20777378.9
(22) Date of filing: 30.03.2020
(51) Int. Cl.: A61K 38/12, A61K 38/05, A61K 38/01, A61P 43/00, A23L 33/18

(54) **USE OF CYCLO-HIS-PRO (CHP) FOR PREVENTING, AMELIORATING, OR TREATING FIBROSIS**

(30) Priority: 28.03.2019 KR 20190036269; 27.03.2020 KR 20200037859
(71) Applicant: Novmetapharma Co., Ltd., Seoul 06050 (KR); Seoul National University Hospital, Seoul 03080 (KR); Seoul National University R&DB Foundation, Gwanak-gu Seoul 08826 (KR)
(72) Inventor: JUNG, Hoe Yune, Pohang-si, Gyeongsangbuk-do 37668 (KR); LEE, Heon Jong, Incheon 21048 (KR); JEON, Jong Su, Pohang-si, Gyeongsangbuk-do 37664 (KR); LEE, Do Hyun, Pohang-si, Gyeongsangbuk-do 37669 (KR); KIM, Yon Su, Seoul 06001 (KR); YANG, Seung Hee, Seoul 04593 (KR); KIM, Yong Chul, Seoul 06547 (KR); MOON, Jong Joo, Seoul 06523 (KR); KIM, Ji Eun, Seoul 03128 (KR)
(74) Representative: Maiwald Patent- und Rechtsanwaltsgesellschaft mbH
(86) International application number: PCT/KR2020/004354
(87) International publication number: WO 2020/197359

(57) **Abstract**

The present invention relates to a use of Cyclo-HisPro (CHP) for preventing, ameliorating, or treating fibrosis and, more particularly, to a pharmaceutical composition for preventing or treating fibrosis comprising CHP, a health functional food composition for preventing or ameliorating fibrosis, an antifibrotic composition, a method of preventing, ameliorating, or treating fibrosis by using CHP, and/or a use of CHP in the manufacture of the pharmaceutical composition for preventing or treating fibrosis.

SEQUENCE LISTING

<110>  NovMetaPharma Co., Ltd.
       SEOUL NATIONAL UNIVERSITY HOSPITAL
       Seoul National University R&DB Foundation

<120>  COMPOSITION COMPRISING CHP (CYCLO-HIS PRO) FOR PREVENTING,
       IMPROVING OR TREATING OF FIBROSIS

<130>  1067299

<150>  KR10-2019-0036269
<151>  2019-03-28

<160>  16

<170>  PatentIn version 3.2

<210>  1
<211>  20
<212>  DNA
<213>  Artificial

<220>
<223>  TGF¥â forward primer

<400>  1
cctgagtggc tgtcttttga                                                   20


<210>  2
<211>  21
<212>  DNA
<213>  Artificial

<220>
<223>  TGF¥â reverse primer

<400>  2
aatcgaaagc cctgtattcc g                                                 21


<210>  3
<211>  20
<212>  DNA
<213>  Artificial

<220>
<223>  Collagen 3 forward primer

<400>  3
agtcaaggag aaagtggtcg                                                   20


<210>  4
<211>  19
<212>  DNA
<213>  Artificial

<220>
<223>  Collagen 3 reverse primer

<400>  4

ccagggaaac ccatgacac 19

<210> 5
<211> 20
<212> DNA
<213> Artificial

<220>
<223> GAPDH forward primer

<400> 5
acactgagca agagagaggc 20

<210> 6
<211> 20
<212> DNA
<213> Artificial

<220>
<223> GAPDH reverse primer

<400> 6
tgggggtctg ggatggaaat 20

<210> 7
<211> 18
<212> DNA
<213> Artificial

<220>
<223> Fibronectin forward primer

<400> 7
ttggagagga gtgggagc 18

<210> 8
<211> 20
<212> DNA
<213> Artificial

<220>
<223> Fibronectin reverse primer

<400> 8
gaaatgacca ctgccaaagc 20

<210> 9
<211> 19
<212> DNA
<213> Artificial

<220>
<223> Collagen 1 forward primer

<400> 9
ctaacgtggt tcgtgaccg 19

<210>  10
<211>  20
<212>  DNA
<213>  Artificial

<220>
<223>  Collagen 1 reverse primer

<400>  10
atccatcggt catgctctct                                                    20


<210>  11
<211>  20
<212>  DNA
<213>  Artificial

<220>
<223>  Collagen 4 forward primer

<400>  11
cggtacacag tcagaccatt                                                    20


<210>  12
<211>  20
<212>  DNA
<213>  Artificial

<220>
<223>  Collagen 4 reverse primer

<400>  12
catcacgaag gaatagccga                                                    20


<210>  13
<211>  18
<212>  DNA
<213>  Artificial

<220>
<223>  ¥â-actin forward primer

<400>  13
gggaaggtga cagcattg                                                      18


<210>  14
<211>  23
<212>  DNA
<213>  Artificial

<220>
<223>  ¥â-actin reverse primer

<400>  14
atgaagtatt aaggcggaag att                                                23


<210>  15
<211>  20
<212>  DNA

<213>  Artificial

<220>
<223>  CTGF forward primer

<400>  15
ctgtgcctgc cattacaact                                                    20


<210>  16
<211>  21
<212>  DNA
<213>  Artificial

<220>
<223>  CTGF reverse primer

<400>  16
ccatgtctcc gtacatcttc c                                                  21

## Description

### [Technical Field]

The present invention relates to a use of Cyclo-HisPro (CHP) for preventing, ameliorating, or treating fibrosis and, more particularly, to a pharmaceutical composition for preventing or treating fibrosis comprising CHP, a health functional food composition for preventing or ameliorating, an antifibrotic composition, a method of preventing, or treating fibrosis by using CHP, and/or a use of CHP in the manufacture of the pharmaceutical composition for preventing or treating fibrosis.

### [Background Art]

Fibrosis is a disease in which abnormal production, accumulation and deposition of extracellular matrix by fibroblasts occurs, and is caused by fibrosis of an organ or tissue. Fibrosis is a very fatal disease that causes long-term damage. For example, idiopathic pulmonary fibrosis (IPF) appears as a result of relapsing alveolar epithelial cell injury associated with fibroblast accumulation and myofibroblast differentiation, and is a chronic, progressive, and lethal disease that causes excessive accumulation of extracellular matrix (ECM) with irreversible destruction of lung parenchyma tissue.

Conventional treatment studies have largely targeted the inflammatory process of fibrosis, using corticosteroids and immunosuppressive drugs. However, these agents show little effect in clinical trials, so there is a need for new drugs for treating fibrosis.

Meanwhile, Korean Patent Laid-Open Publication No. 10-2013-0006170 discloses a composition for blood sugar regulation comprising soybean hydrolysate with high CHP (CYCLO(His-Pro), but the antifibrotic effect of CHP is not known.

### [Disclosure of Invention]

### [Technical Problem]

One aspect of the present invention is directed to providing a pharmaceutical composition for preventing or treating fibrosis comprising Cyclo-HisPro.

Another aspect of the present invention is directed to providing a health functional food composition for preventing or ameliorating fibrosis comprising Cyclo-HisPro.

Another aspect of the present invention is directed to providing an antifibrotic composition comprising Cyclo-HisPro.

Another aspect of the present invention is directed to providing a method of preventing, or treating fibrosis by using Cyclo-HisPro.

Another aspect of the present invention is directed to providing a use of Cyclo-HisPro in the manufacture of the pharmaceutical composition for preventing or treating fibrosis.

### [Technical Solution]

In order to solve the above problems, the present invention provides a pharmaceutical composition for preventing or treating fibrosis comprising Cyclo-HisPro or a pharmaceutically acceptable salt thereof.

In addition, the present invention provides a health functional food composition for preventing or ameliorating fibrosis comprising Cyclo-HisPro or a sitologically acceptable salt thereof.

Further, the present invention also provides a method for preventing or treating fibrosis comprising administering to a subject in need thereof an effective amount of Cyclo-HisPro.

The present invention also provides a use of Cyclo-HisPro in the manufacture of a pharmaceutical composition for preventing or treating fibrosis.

According to a preferred embodiment of the present invention, the fibrosis may develop in any one or more selected from the group consisting of: kidney, liver, lung, skin, heart, pancreas, urinary system, genital system, sweat gland, nerve, brain, bone marrow, muscle and joint.

According to another preferred embodiment of the present invention, the fibrosis may be any one or more selected from the group consisting of: pulmonary fibrosis, idiopathic pulmonary fibrosis, radiation-induced lung injury or lung fibrosis, pulmonary edema, cystic fibrosis, liver fibrosis, endomyocardial fibrosis, myocardial infarction, artrial fibrosis, glial scar, renal fibrosis, myelofibrosis, arthrofibrosis, fat fibrosis, skin fibrosis, neurofibrosis and myofibrosis.

The present invention also provides an antifibrotic composition comprising Cyclo-HisPro.

### [Advantageous Effects]

The composition comprising Cyclo-HisPro of the present invention is effective for preventing, ameliorating or treating fibrosis by inhibiting fibrosis occurring in various tissues or organs such as kidney, liver, lung, skin, heart, pancreas, urinary system, genital system, sweat glands, nerves, brain, bone marrow, muscles and joints.

### [Brief Description of Drawings]

The above and other aspects, features, and advantages of the present invention will become more apparent to those of ordinary skill in the art by describing embodiments thereof in detail with reference to the accompanying drawings, in which:
FIG. 1 shows the process of cell isolation and primary culture from human glomeruli and renal tubules (left: glomerulus, right: tubule);
FIG. 2 shows the morphological changes of cells according to CHP treatment (4, 20 and 100 µg/ml, respectively) by concentration in a human-derived proximal tubule epithelial cell fibrosis model;
FIG. 3 shows a result of confirming changes in the expression level of E-cadherin, fibronectin and pSTAT3 proteins by Western blot according to CHP treatment (4, 20 and 100 µg/ml, respectively) by concentration in a human-derived proximal tubule epithelial cell fibrosis model;
FIG. 4 shows the morphological changes of cells according to CHP treatment (40 and 100 µg/ml, respectively) by concentration in a human-derived glomerular endothelial cell fibrosis model;
FIG. 5a shows a band result of confirming changes in the expression level of fibronectin protein by Western blot according to CHP treatment (62.5, 125 and 250 ng/ml, respectively) by concentration in a human-derived liver cell fibrosis model, and FIG. 5b is a graph of quantifying the size of the band;
FIG. 6a shows a band result of confirming changes in the expression level of fibronectin protein by Western blot according to CHP treatment (5 and 35 mg/kg, respectively) by concentration in an animal model of liver fibrosis, and FIG. 6b is a graph of quantifying the size of the band;
FIG. 7a shows a band result of confirming changes in the expression level of fibronectin protein by Western blot according to CHP treatment (62.5 and 125 ng/ml, respectively) by concentration in a human-derived lung cell fibrosis model, and FIG. 7b is a graph of quantifying the size of the band;
FIG. 8 is a graph showing changes in the expression level of TGFβ and *Collagen* 3 genes according to CHP administration (5 and 35 mg/kg, respectively) by concentration in an animal model of lung fibrosis;
FIG. 9a shows a band result of confirming changes in the expression level of fibronectin protein by Western blot according to CHP treatment (62.5, 125, 250 and 500 ng/ml, respectively) by concentration in a human-derived skin cell fibrosis model, and FIG. 9b is a graph of quantifying the size of the band;
FIG. 10 is a graph showing changes in the expression level of fibrosis markers TGFβ, *Fibronectin, Collagen 1, Collagen 2, Collagen 3* and *Collagen 4* genes according to CHP administration (5 and 35 mg/kg, respectively) by concentration in an animal model of cardiac fibrosis;
FIG. 11 is a graph showing changes in the expression level of fibrosis markers TGFβ, *Fibronectin, Collagen 3*, 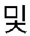 *CTGF* genes according to CHP administration (5 and 35 mg/kg, respectively) by concentration in an animal model of fat fibrosis.

### [Best Mode for Carrying Out the Invention]

Hereinafter, the present invention will be described in more detail.

As described above, conventional fibrosis-related treatment studies have largely targeted the inflammatory process of fibrosis, using corticosteroids and immunosuppressive drugs, but these agents show little effect in clinical trials, so there is a need for new drugs for treating fibrosis.

Accordingly, the present inventors confirmed that Cyclo-HisPro is effective in preventing, ameliorating, or treating fibrosis by inhibiting fibrosis occurring in various tissues or organs, and completed the present invention.

Accordingly, the present invention provides a pharmaceutical composition for preventing or treating fibrosis comprising Cyclo-HisPro or a pharmaceutically acceptable salt thereof and/or a health functional food composition for preventing or ameliorating fibrosis comprising Cyclo-HisPro or a sitologically acceptable salt thereof.

The present invention also provides an antifibrotic composition comprising Cyclo-HisPro.

In the present invention, "Cyclo-HisPro (CHP)" is a naturally occurring circular dipeptide composed of histidine-proline which is a metabolite of thyrotropin-releasing hormone (TRH) or a physiologically active dipeptide that is also synthesized in the body through the TRH metabolic process and de novo, and refers to a substance widely distributed throughout the brain and in spinal cord and gastrointestinal tract.

In the composition of the present invention, the CHP may be synthesized or commercially available. In addition, it can be used after purification from substances containing CHP, for example, prostate extract and soybean hydrolysate.

By use of the term "purified", it is intended to mean that CHP is in a concentrated form compared to a form obtainable from a natural origin, such as a prostate extract. Purified ingredients can be concentrated from their natural sources or obtained through chemical synthesis methods.

As used herein, the term "fibrosis" is used interchangeably with "fibrotic condition", "fibroproliferative condition", "fibrotic disease", "fibroproliferative disease", "fibrotic disorder" and "fibroproliferative disorder", and refers to a condition, disease or disorder characterized by dysregulated proliferation or activity of fibroblasts, abnormal accumulation of fibronectin and/or pathological or excessive accumulation of collagenous tissue. Typically, such conditions, diseases or disorders are treatable by administration of a compound having antifibrotic activity.

The fibrosis may develop, for example, in any one or more selected from the group consisting of: kidney, liver, lung, skin, heart, pancreas, urinary system, genital system, sweat gland, nerve, brain, bone marrow, muscle and joint.

For example, the fibrosis of the present invention may be any one or more selected from the group consisting of: pulmonary fibrosis, idiopathic pulmonary fibrosis, radiation-induced lung injury or lung fibrosis, pulmonary edema, cystic fibrosis, liver fibrosis, endomyocardial fibrosis, myocardial infarction, artrial fibrosis, glial scar, renal fibrosis, myelofibrosis, arthrofibrosis, fat fibrosis, skin fibrosis, neurofibrosis and myofibrosis, but is not limited thereto.

Specifically, the CHP according to the present invention has therapeutic effects on the various fibrosis described herein.

In the use of the composition of the present invention for preventing, ameliorating or treating fibrosis, a first aspect relates to renal fibrosis, which is fibrosis occurring in the kidney.

Kidney disease is classified into acute renal failure or chronic renal failure depending on the progression status, or depending on the cause of disease, divided into glomerulonephritis due to deposition of vascular complexes, diabetic kidney disease accompanying diabetes or hypertensive renal disease accompanying hypertension, toxic nephropathy caused by drug administration such as antibiotics or anticancer agents, bacterial infection, and the like. Regardless of the causative kidney disease, if the glomerular filtration rate is reduced to 50% or less due to chronic renal dysfunction, in most cases, the glomerular filtration rate continues to decrease, ultimately leading to end-stage renal failure, developing complications such as hematologic abnormalities, nervous system complications, gastrointestinal complications, immunological complications, infections, or osteodystrophy, and in severe cases, leading to death.

Chronic renal failure is a state in which the renal function gradually decreases in one direction (irreversibly), and the homeostasis of the living body cannot be maintained. All kidney diseases involve fibrosis of the kidneys, eventually leading to end-stage renal failure. In particular, since chronic renal function decline is deeply related to the progression of renal fibrosis, inhibition of the progression of fibrosis may lead to inhibition of the progression of chronic renal failure.

As used herein, the term "renal fibrosis" includes all diseases in which fibrosis occurs in the kidney due to various causes, and the fibrosis may include, but is not limited to, those caused by any one or more selected from the group consisting of: catheter installation, glomerulosclerosis, glomerulonephritis, nephritis, acute renal failure, chronic renal failure, end-stage renal disease, and metabolic disease.

The type of nephritis may include, but is not limited to, for example, any interstitial nephritis, for example, lensacoccal (Streptococcus sp.) nephritis, staphylococcal nephritis; viral nephritis accompanying alastrim, hepatitis B, hepatitis C, HIV, etc.; nephritis caused by parasitic infections such as malaria; fungal nephritis, infectious interstitial nephritis accompanying mycoplasma nephritis, systemic erythematosus (lupus nephritis), systemic scleroderma (collagen disease kidney), interstitial nephritis accompanying collagen diseases such as Sjgren's syndrome, purpura nephritis, polyarteritis, nephritis accompanying vascular immune diseases such as rapidly progressive glomerulonephritis, interstitial nephritis accompanying radiation exposure; drug-induced interstitial nephritis caused by anticancer drugs such as gold agents, NSAID, penicillamine, and bleomycin, antibiotics, paraquat, etc.; allergic nephritis caused by insect bites, pollen, or rhus plants, etc.; nephritis accompanying amyloidosis nephritis, diabetic renal failure, chronic glomerulonephritis, malignant nephrosclerosis, polycystic kidney failure, etc.; tubulointerstitial nephritis, nephritis accompanying pregnancy toxaemia or cancer; idiopathic interstitial nephritis such as membranous proliferative glomerulonephritis, IgArenal failure, mixed cryoglobulinemia nephritis, Goodpasture's-syndrome nephritis, beguena granulomatous nephritis, acute interstitial nephritis.

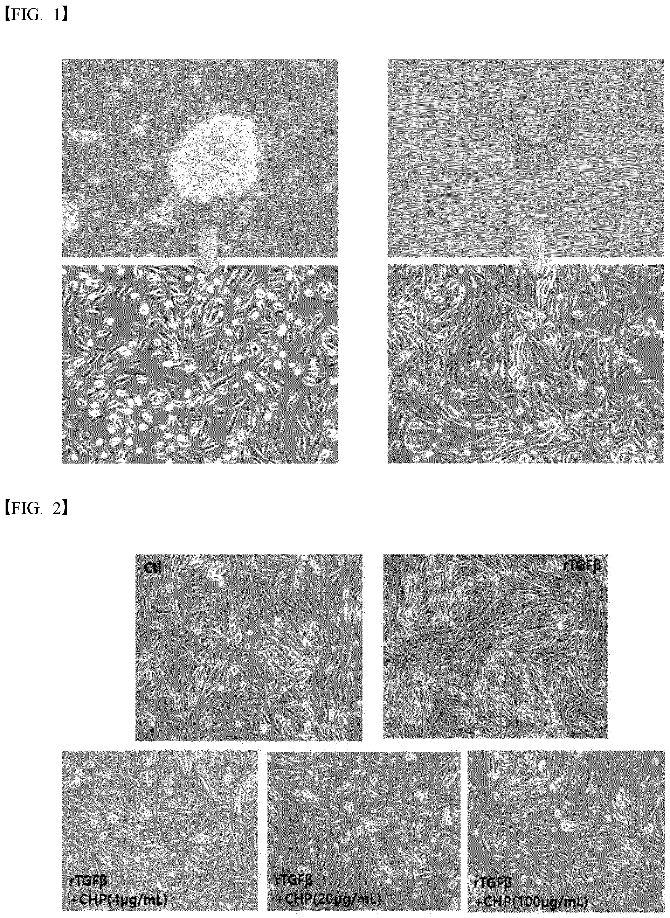

As demonstrated in FIG. 2 to 4, CHP has a fundamental antifibrotic effect by restoring the expression of the splicing marker E-cadherin protein and reducing the expression of fibronectin and pSTAT3 protein in the proximal tubular epithelial cells induced by fibrosis, and therefore, regardless of the cause that induces fibrosis, it can be applied to treat various renal fibrosis.

In the use of the composition of the present invention for preventing, ameliorating or treating fibrosis, a second aspect relates to liver fibrosis, which is fibrosis occurring in the liver.

As used herein, the term "liver fibrosis" refers to a symptom in which fibrous tissue proliferates due to chronic damage to the liver, and may include, but is not limited to, those caused by any one or more selected from the group consisting of: chronic liver disease, hepatitis B virus infection, hepatitis C virus infection, hepatitis D virus infection, schistosomiasis, alcoholic liver disease or non-alcoholic steatohepatitis, metabolic disease, protein deficiency, coronary artery disease, autoimmune hepatitis, cystic fibrosis, alpha-1 antitrypsin deficiency, primary biliary cirrhosis, drug reactions and toxins.

Liver fibrosis is a precursor to cirrhosis and is initiated by the action of various cytokines and growth factors as a result of severe liver damage, leading to chronic liver disease. In general, liver fibrosis consists of reversible and thin fibrils, and when there is no nodule formation and the cause of liver damage is temporary, the extracellular matrix (ECM) increased by the process of apoptosis and matrix metalloproteinases (MMP) is decomposed, and normal recovery is possible, but if the liver fibrosis process continues repeatedly, it forms thick fibrils and progresses to nodular cirrhosis. In addition, liver cirrhosis is induced through the process of liver fibrosis, in which hepatocytes are damaged by various inflammatory factors and so abnormal extracellular matrix proteins including collagen are accumulated, and it is important to control the accumulation of extracellular matrix to control the expression of cirrhosis. Inflammatory response when hepatocytes are damaged activates resting hepatic stellate cells to secrete extracellular matrix and various cytokines and chemokines, among which TGF-β1 acts as a strong growth inhibitory agent. TGF-β1 is a 25 kD substance, which binds to the latent TGF-β1 binding protein and is secreted in the form of an inactive latent, and it exists in a state combined with extracellular matrix such as type 1 and 4 collagen, laminin and decorin, and is activated by various stimuli. TGF-β1 regulates collagen expression by decreasing the production of collagenase or increasing the production of collagenase inhibitors, increases the production of TNF-α, IL-1 and PDGF in macrophages, and play an important role in the fibrosis process. Currently, it is known that TGF-β1 plays an important role in liver fibrosis, since TGF-β1 is expressed only in areas where fibrosis has progressed and is not expressed in normal liver tissues or inactive areas.

Meanwhile, non-alcoholic fatty liver can be caused by causes such as obesity, diabetes, hyperlipidemia, drugs, etc. regardless of drinking alcoholic, and depending on the progress, refers to a wide range of diseases including from simple steatosis without inflammatory response to non-alcoholic steatohepatitis (NASH) showing hepatocellular inflammatory response, advanced fibrosis and liver cirrhosis.

In the case of nonalcoholic fatty liver disease (NAFLD), it has been reported that due to the increase in adult diseases caused by high-fat and high-calorie diets in modern society, 20-30% of the adult population in developed countries develop nonalcoholic fatty liver disease (NAFLD), and 2-3% of them transition to nonalcoholic steatohepatitis (NASH) patients, and in particular, they show histological findings of steatohepatitis accompanied by fibrosis and inflammation, highly increasing the risk of developing cirrhosis, liver failure, and liver cancer.

Accordingly, in an embodiment of the present invention, an animal model of liver fibrosis induced by a high-fat diet was established, and it was confirmed that CHP exhibits the effect of reducing fibronectin protein expression in the model.

Fatty liver is not a pathological condition in itself, and is a reversible symptom that naturally recovers when the causative substance is removed. However, if the state of excessive fat accumulation in the liver tissue is continuously maintained, steatohepatitis occurs, and as a result, hepatocyte necrosis and regeneration occur repeatedly, during which the fibrous extracellular matrix (ECM) increases, leading to liver fibrosis. When liver damage reaches a certain stage, the accumulation of ECM increases regardless of the type of causative agent, and as a result of continuous destruction and regeneration of hepatocytes, regenerative nodules are formed, which worsens into irreversible liver cirrhosis.

Therefore, in the present invention, liver fibrosis is a disease clearly distinguished from fatty liver, which is a reversible symptom, and refers to any disease in which liver function is reduced by changing liver tissue to fibrotic tissue such as regenerative nodules.

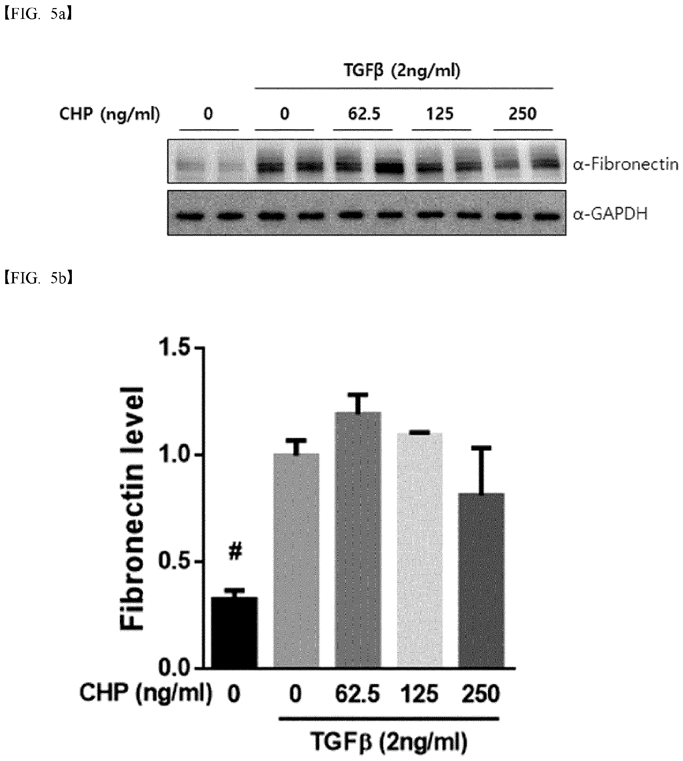

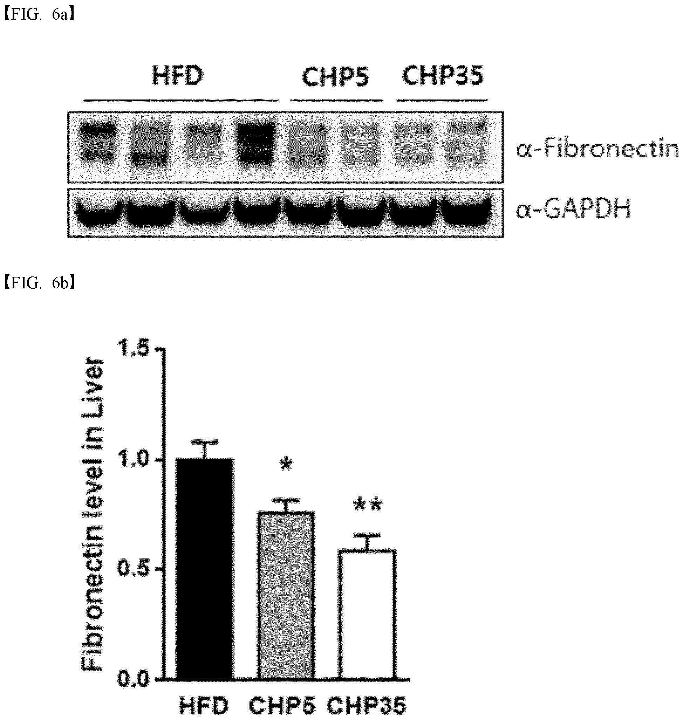

As demonstrated in FIG. 5, CHP reduces the expression level of fibronectin protein, a fibrotic protein, in fibrosis-induced liver cells, and as demonstrated in FIG. 6, CHP reduces the expression of fibronectin protein in an animal model in which liver fibrosis is induced, thereby exhibiting a fundamental antifibrotic effect, so it can be applied to the treatment of liver failure and liver cancer that have progressed from cirrhosis, as well as various liver fibrosis, regardless of the cause of fibrosis.

In the use of the composition of the present invention for preventing, ameliorating or treating fibrosis, a third aspect relates to pulmonary fibrosis, which is fibrosis occurring in the lung.

Especially among fibrosis, pulmonary fibrosis refers to a disease in which chronic inflammatory cells infiltrate into the alveolar wall of the lung tissue and induce tissue fibrosis to cause serious structural changes in the lung tissue. Once fibrosis is progressed due to any cause, the lung tissue hardens and the alveolar wall thickens, thereby reducing the amount of oxygen supplied by the blood, which makes breathing difficult. Currently, there is no treatment method that can completely restore the lung tissue that has already progressed to fibrosis, so if it is detected at an early stage of fibrosis or except for lung transplantation, usually 3 to 5 years after the onset of symptoms, the patient will eventually die.

Specifically, as used herein, the term "pulmonary fibrosis" refers to the development of scarred (fibrous) tissue due to the formation or development (fibrosis) of excessive fibrous connective tissue in the lung. Specifically, pulmonary fibrosis is a chronic disease that causes swelling and scarring of the alveoli and interstitial tissues of the lungs. Such scar tissue replaces healthy tissue and causes inflammation, and chronic inflammation can be identified as a precursor of fibrosis. Due to such damage to the lung tissue, the lungs may become stiff, and it may become difficult for the individual to breathe on their own.

Pulmonary fibrosis in the present invention may include, but is not limited to, any one or more selected from the group consisting of: idiopathic pulmonary fibrosis, radiation-induced lung injury, nonspecific interstitial pneumonia, acute interstitial pneumonia, respiratory bronchiolitis associated interstitial lung, desquamative interstitial pneumonia, lymphoid interstitial pneumonia, interstitial pulmonary fibrosis, diffuse pulmonary fibrosis, pulmonary edema, cystic fibrosis, and pulmonary fibrosis due to metabolic disease.

The pulmonary fibrosis may be caused by various causes, for example, microscopic damage to the lungs induced by inhalation of fine particles (asbestos, stone dust, metal dust, particles present in cigarette smoke, silica dust, etc.). In addition, pulmonary fibrosis may also occur as a secondary effect of other diseases (such as autoimmune diseases, viral or bacterial infections) and may be caused by certain drugs such as cytotoxic agents (such as bleomycin, busulfan and methotrexate); antibiotics (such as nitrofurantoin and sulfasalazine); antiarrhythmic drugs (such as amiodarone and tocainide); anti-inflammatory drugs (such as gold and penicylamine); illegal drugs (such as narcotics, cocaine and heroin), and the idiopathic pulmonary fibrosis may be caused by other unknown causes other than causes above. In addition, long-term intake of high-fat diet induces pulmonary fibrosis, which appears to be due to increased inflammatory levels following high-fat intake.

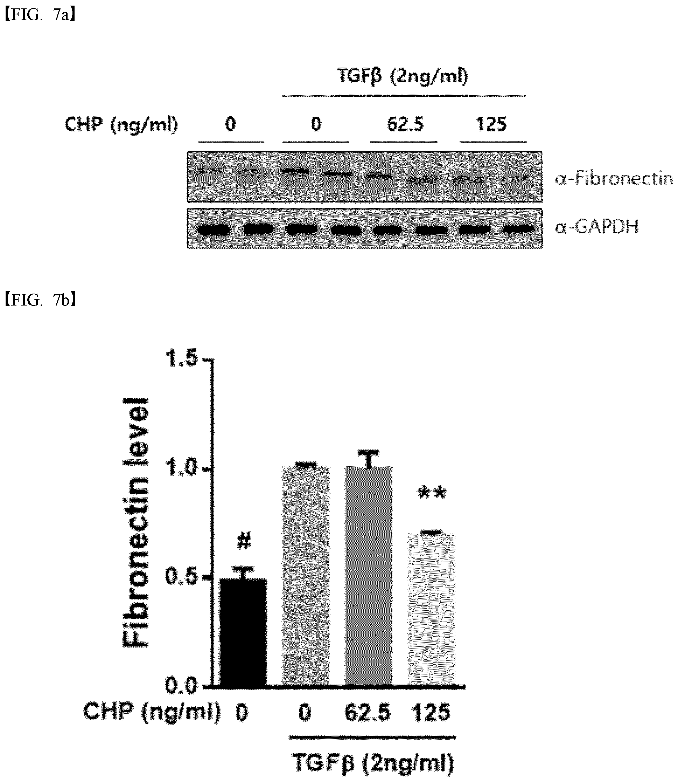

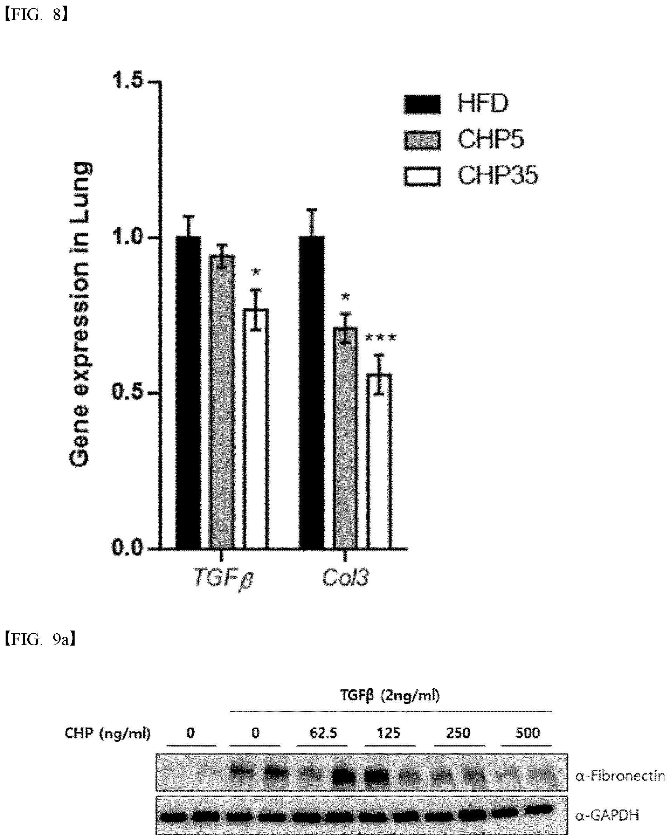

As demonstrated in FIG. 7, CHP reduces the expression level of fibronectin protein, a fibrotic protein, in fibrosis-induced lung cells, and as demonstrated in FIG. 8, CHP reduces the expression of TGFβ and *Collagen 3* genes, which are major fibrosis markers, in a pulmonary fibrosis-induced animal model, thereby exhibiting a fundamental antifibrotic effect, and therefore, regardless of the cause that induces fibrosis, it can be applied to treat various pulmonary fibrosis.

In the use of the composition of the present invention for preventing, ameliorating or treating fibrosis, a fourth aspect relates to skin fibrosis.

As used herein, the term "skin fibrosis" is excessive scarring on the skin and is a result of a pathological wound healing response. There is a wide range of fibrotic skin diseases: scleroderma, renal fibrosing dermatosis, mixed connective tissue disease, scleromyxedema, sclerosing edema, and eosinophilic fasciitis. Exposure to chemicals or physical agents (for mechanical trauma, burn wounds) is also a potential cause of fibrotic skin disease. Skin fibrosis may be driven by immune, autoimmune and inflammatory mechanisms. The balance of collagen production and degradation in fibroblasts plays an important role in the pathophysiological process of skin fibrosis. Certain cytokines, such as transforming growth factor-β (TGF-β) and interleukin-4 (IL-4) promote wound healing and fibrosis. Normal skin fibroblasts are stationary. They control the amount of connective tissue protein and have low proliferative activity. After skin injury, these cells are activated, that is, they express α-smooth muscle actin (a-SMA) and synthesize large amounts of connective tissue protein. Such activated cells are often called myofibroblasts.

As used herein, the term "skin fibrosis" is also meant to include "scleroderma".

Scleroderma is a chronic autoimmune connective tissue disease of unknown cause characterized by sclerotic changes and vascular abnormalities in which the skin of a part or the whole body becomes hard and thickened due to excessive accumulation of collagen in the dermis. Collagen forms connective tissue and serves to support and connect the tissues of the body. There are several forms of scleroderma, some showing symptoms only in a specific part of the body, while others appearing all over the body including internal organs. That is, it is divided into localized scleroderma, in which only a part of the skin becomes hard, and systemic scleroderma, which exhibits fibrosis symptoms due to the increase in collagen not only in the skin but also in internal organs such as the lungs, digestive organs, kidneys and heart. Systemic scleroderma is further classified into limited type and diffuse type according to the degree of invasion of the skin and internal organs, prognosis, and immunological examination findings.

In the use of the present invention for preventing, ameliorating or treating skin fibrosis, skin fibrosis may include, but is not limited to, any one or more selected from the group consisting of: scars, hypertrophic scars, keloid scars, localized scleroderma and systemic scleroderma.

Although the cause of scleroderma has not yet been clearly identified, tissue fibrosis is thought to play an important role.

The early symptoms of scleroderma are very diverse, especially in the latter stage, the skin symptoms become clear. Common symptoms of scleroderma include joint pain, morning stiffness, fatigue, and weight loss. In addition, exposure to cold also temporarily limits the blood supply to fingers, toes, nose and ears. These symptoms are early symptoms of scleroderma patients and are one of the frequent symptoms. The skin of patients with scleroderma becomes hard. This hardening of the skin spreads widely and typically occurs on both sides of the body. Eventually, the tissue is damaged, and the skin is pigmented.

"Localized scleroderma" is 2.6 times more common in women than in men. Localized scleroderma occurs in 75% of patients between 20 and 50 years of age, and linear scleroderma tends to occur at an earlier age. Localized scleroderma is classified into localized scleroderma, systemic localized scleroderma, linear localized scleroderma, or subcutaneous localized scleroderma.

Localized scleroderma often starts with erythema or reddish-purple patches, and the border with the surrounding normal skin becomes clear, and the skin in a certain area loses elasticity and becomes hard. The area will be brown or, in many cases, discolored, giving it a white color. Most of the time it occurs in one place, but sometimes there are several, and they vary in size and can range from the size of a coin to the size of an adult palm. It causes cosmetic problems but does not progress to systemic scleroderma. In some patients, even without treatment, the hard skin softens and recovers on its own.

Systemic localized scleroderma is a severe form of localized scleroderma, in which the skin in a large area becomes hard and hyperpigmentation is observed. Extensive hardening of the skin of the trunk, buttocks, and legs. However, it is distinguished from systemic sclerosis because there is no Raynaud's phenomenon or internal organ involvement.

Linear localized scleroderma is a case in which the skin becomes long and hard in a straight line, and it occurs most often in the order of the legs, arms, forehead, and chest, in particular, if it is depressed in a vertical line on the forehead, it is called en coup de sabre and causes cosmetic problems. Unlike localized scleroderma, linear scleroderma involves not only the skin but also the underlying muscle and periosteum tissue and is fixed in the underlying tissue. It occurs more frequently in children and can sometimes cause severe skeletal developmental anomaly in the limbs and face.

Subcutaneous localized scleroderma is characterized by sclerosis of the fat layer, fascia, muscle and sometimes bone, and joint movement may be limited. Because the lesion appears in the deep part, the characteristic skin pigment change of scleroderma is not observed.

Systemic scleroderma occurs four times more often in women than in men, and can occur at any age, but usually occurs between the ages of 30 and 50. The diffuse type is known to occur at a younger age than the limited type. Raynaud's phenomenon is the first symptom of systemic scleroderma, followed by sclerosis of the skin and internal organs. Systemic scleroderma is divided into skin symptoms and internal organ symptoms.

In systemic sclerosis, the first skin symptoms begin on the fingers and hands. At first, only the Raynaud's phenomenon is seen, but the fingers and hands become swollen, stiff, and turn red. Gradually, the skin becomes hard, and it spreads to the arms and face, and in the case of the diffuse type, the skin of the whole body including the trunk is continuously hardened. When sclerosis occurs on the face, the overall wrinkles disappear, and it becomes difficult to make expressions. The nose looks pointed, it is difficult to open the mouth, and the lips become thin, and radial wrinkles around the mouth appear, making it look like an elderly person's mouth. The movement of the joint is limited, making it impossible to hold the hand all the way, and sclerodactyly appears in which the fingers are bent and pointed. Painful and difficult-to-heal ulcers develop on the fingertips and joints.

Extensive hyperpigmentation and local hypopigmentation may occur, and hair loss and sweat secretion reduction occur in the involved area. Capillary dilatation appears in the form of round spots on the face and upper body, or is observed around the nail folds. If there is an abnormality in the capillaries of the nail folds in patients with Raynaud's disease, systemic scleroderma may occur later, so changes in capillary are important in determining the prognosis. Skin calcification can be observed and is relatively common around the finger joints.

Internal organ symptoms occur in the gastrointestinal tract, lungs, heart, or kidneys. In the gastrointestinal tract, the esophagus is the most frequently involved part and is involved in more than 90% of cases. In the esophagus, peristaltic dysfunction, dysphagia, and reflux esophagitis are common. Symptoms such as constipation, diarrhea, and malabsorption may appear due to decreased intestinal motility. The most important cause of death in patients with systemic scleroderma is due to pulmonary symptoms, occurring in about 70% of patients. Due to fibrosis of the lungs, people complain of difficulty in breathing and cough during exercise. Alveolitis is a common occurrence, and pulmonary function continues to decline. Cardiac symptoms may include cardiac conduction disorder, heart failure, and pericarditis, and myocardial sclerosis may occur in 50-70% of patients. Kidney symptoms occur in approximately 45% of patients with systemic scleroderma. It appears as sudden onset of acute renal failure, hypertension, etc., and uremia that progresses slowly may occur.

Accordingly, in the use of the present invention for preventing, ameliorating or treating skin fibrosis, skin fibrosis encompasses, without limitation, fibrosis occurring in any part of: internal cavities of organs or glands such as blood vessels and veins, ducts of submandibular, gallbladder, thyroid follicles, sudoriferous ducts, ovaries, kidneys; epithelial cells of the gums, tongue, palate, nose, larynx, esophagus, stomach, intestines, rectum, anus and vagina; and any skin tissue and epithelial cells, including the dermis, scars, skin and scalp.

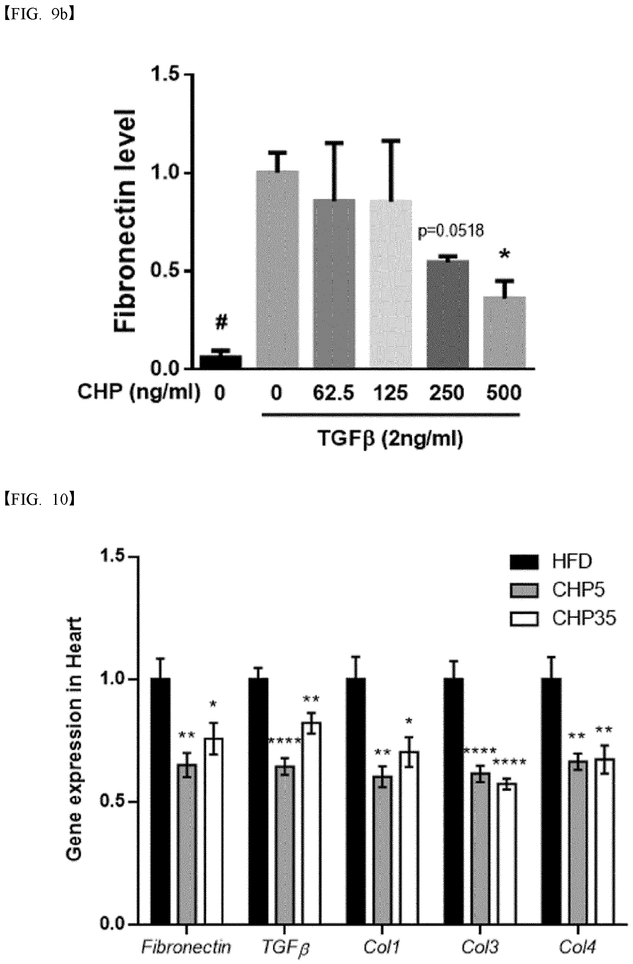

As confirmed in FIG. 9, CHP exhibits a fundamental antifibrotic effect by reducing the expression level of fibronectin protein, a fibrotic protein, in fibrosis-induced skin cells, and therefore, regardless of the cause that induces fibrosis, it can be applied to treat various skin fibrosis (scleroderma).

In the use of the composition of the present invention for preventing, ameliorating or treating fibrosis, a fifth aspect relates to fibrosis, which is cardiac fibrosis occurring in the heart.

As used herein, the term "cardiac fibrosis" refers to a phenomenon in which the heart hardens due to excessive deposition of matrix proteins between heart cells and also refers to a phenomenon that occurs mainly in the heart of patients with myocardial infarction and is the main cause of decreased heart function, and may include, but not limited to, any one or more selected from the group consisting of: endomyocardial fibrosis, atrial fibrosis, heart failure, myocardial infarction, and cardiac fibrosis due to metabolic disease.

Cardiac fibrosis is characterized by a disproportionate accumulation of fibrosing collagen that occurs after cardiomyocyte death, inflammation, increased workload, hypertrophy and stimulation by a number of hormones, cytokines and growth factors.

Cardiac fibrosis may also refer to abnormal thickening of heart valves due to disproportionate proliferation of cardiac fibroblasts, but more generally refers to proliferation of fibroblasts in the heart muscle. Fibrocyte cells normally act to secrete collagen and provide structural support for the heart. When overactivated, this process leads to valve thickening and fibrosis.

Since fibrosis of the heart is a major cause of heart failure and myocardial infarction, the term "cardiac fibrosis" may be interpreted to encompass heart failure and/or myocardial infarction caused by cardiac fibrosis.

As confirmed in FIG. 10, CHP exhibits a fundamental antifibrotic effect by reducing the expression of *Fibronectin, Collagen 1, Collagen 2, Collagen 3* and *Collagen* 4 genes, which are major fibrosis markers, in a fibrosis-induced animal model, and therefore, regardless of the cause that induces fibrosis, it is useful for the treatment of various cardiac fibrosis.

In the use of the composition of the present invention for preventing, ameliorating or treating fibrosis, a sixth aspect relates to fat fibrosis.

As used herein, the term "fat fibrosis" is meant to encompass all diseases in which fibrosis occurs in adipose tissue.

It has been reported that fibrosis of adipose tissue occurs when normal mice are fed a high-fat diet containing 60% fat for 16 to 24 weeks (Hu, M et al., Evidence-Based Complementary and Alternative Medicine, 1-12, 2018; Kwon, E. Y, & Choi, M. S., Nutrients, 10(10), 1415, 2018; Nakazeki, F et al., Scientific Reports, 8(1), 2018; Muniappan, L et al. Scientific Reports, 7(1), 2017; Lancha, A et al., PLoS ONE, 9(5), e98398, 2014; Velαzquez, K. T et al., Physiological Reports, 5(18), e13412, 2017; Wang, L., Ye, X., Hua, Y, & Song, Y., Biomedicine & Pharmacotherapy, 105, 121-129, 2018).

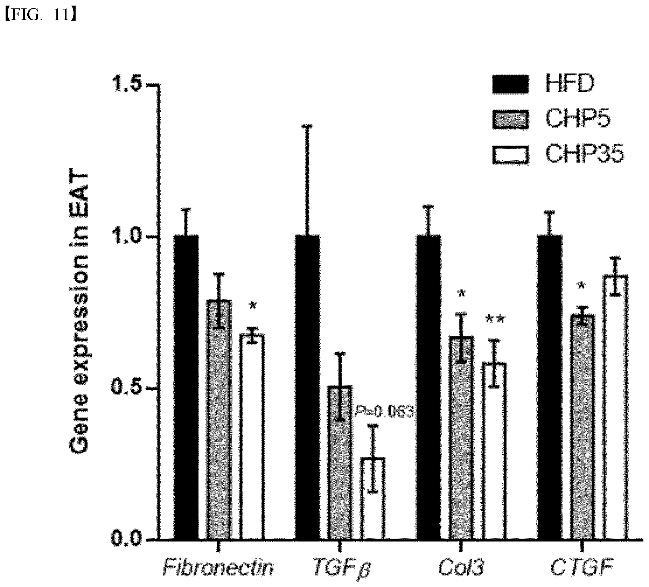

Accordingly, the present inventors established an animal model of fat fibrosis induced by a high-fat diet and checked the expression changes of the fibrosis markers TGFβ, *Fibronectin, Collagen* 3, and *CTGF* genes according to CHP administration. As a result, as confirmed in FIG. 11, CHP exhibits an antifibrotic effect by remarkably reducing the expression of the fibrosis markers, and thus is useful for the treatment of fat fibrosis.

As described above, in the present invention, it was confirmed that CHP exhibits antifibrotic activity by reducing the expression of various fibrosis marker genes or proteins in various fibrosis-induced tissue cells and animal models, therefore, it can be expected that it can be effectively applied to prevent, ameliorate, or treat fibrosis occurring in various tissues and/or organs in the body, including kidney, liver, lung, skin, heart, pancreas, urinary system, genital system, sweat glands, nerves, brain, bone marrow, muscles and joints.

In the composition for preventing, ameliorating or treating fibrosis of the present invention, the terms "prevent", "ameliorate" and/or "treat" refer to any act that inhibits or delays the onset of a disease or disease symptom, any act that ameliorates or beneficially alters the state of a disease or disease symptom, and any act that delays, stops or reverses the progression of a disease or disease symptom.

As used herein, the term "pharmaceutically acceptable" refers to those that are physiologically acceptable and do not normally cause allergic reactions or similar reactions when administered to humans, and the salt is preferably an acid addition salt formed by a pharmaceutically acceptable free acid.

The pharmaceutically acceptable salt may be an acid addition salt formed using an organic acid or an inorganic acid, and the organic acid include, for example, formic acid, acetic acid, propionic acid, lactic acid, butyric acid, isobutyric acid, trifluoroacetic acid, malic acid, maleic acid, malonic acid, fumaric acid, succinic acid, succinic acid monoamide, glutamic acid, tartaric acid, oxalic acid, citric acid, glycolic acid, glucuronic acid, ascorbic acid, benzoic acid, phthalic acid, salicylic acid, anthranilic acid, dichloroacetic acid, aminooxy acetic acid, benzenesulfonic acid, p-toluenesulfonic acid or methanesulfonic acid. The inorganic acid includes, for example, hydrochloric acid, bromic acid, sulfuric acid, phosphoric acid, nitric acid, carbonic acid or boric acid. The acid addition salt may preferably be in the form of hydrochloride salt or acetate salt, and more preferably in the form of hydrochloride salt.

In addition, additional salts are available in the form of GABA salt, gabapentin salt, pregabalin salt, nicotinate salt, adipate salt, hemimalonate salt, cysteine salt, acetylcysteine salt, methionine salt, arginine salt, lysine salt, ornithine salt or aspartate salt etc.

In addition, the pharmaceutical composition of the present invention may further include a pharmaceutically acceptable carrier. The pharmaceutically acceptable carrier may further include, for example, a carrier for oral administration or a carrier for parenteral administration. Carriers for oral administration may include lactose, starch, cellulose derivatives, magnesium stearate, stearic acid, and the like. Carriers for parenteral administration may include water, suitable oils, saline, aqueous glucose and glycols, and the like. In addition, it may further include a stabilizer and a preservative. Suitable stabilizers include antioxidants such as sodium bisulfite, sodium sulfite or ascorbic acid. Suitable preservatives include benzalkonium chloride, methyl- or propyl-paraben and chlorobutanol. As to other pharmaceutically acceptable carriers, reference may be made to those described in the following literature (Remington's Pharmaceutical Sciences, 19th ed., Mack Publishing Company, Easton, PA, 1995).

The pharmaceutical composition of the present invention may be administered to mammals, including humans by any method. For example, it may be administered orally or parenterally, and parenteral administration methods include, but are not limited to, intravenous, intramuscular, intraarterial, intramedullary, intradural, intracardiac, transdermal, subcutaneous, intraperitoneal, intranasal, enteral, topical, sublingual or rectal administration.

The pharmaceutical composition of the present invention may be formulated as a formulation for oral administration or parenteral administration according to the administration route as described above. When formulated, it may be prepared using one or more buffers (e.g., saline or PBS), carbohydrates (e.g., glucose, mannose, sucrose, or dextran, etc.), antioxidants, bacteriostatic agents, chelating agents (e.g., EDTA or glutathione), fillers, extending agents, binding agents, adjuvants (e.g. aluminum hydroxide), suspending agents, thickening agents, wetting agents, disintegrating agents or surfactants, diluents or excipients.

Solid formulations for oral administration include tablets, pills, powders, granules, liquids, gels, syrups, slurries, suspensions or capsules, etc., and these solid formulations may be prepared by mixing the pharmaceutical composition of the present invention with at least one or more excipients, for example, starch (including corn starch, wheat starch, rice starch, potato starch, etc.), calcium carbonate, sucrose, lactose, dextrose, sorbitol, mannitol, xylitol, erythritol maltitol, cellulose, methyl cellulose, sodium carboxymethyl cellulose and hydroxypropylmethyl-cellulose or gelatin, etc. For example, tablets or sugar-coated tablets may be obtained by blending the active ingredient with solid excipients, grinding them, adding suitable adjuvants, and processing them into a granular mixture.

In addition to simple excipients, lubricants such as magnesium stearate talc are also used. Liquid formulations for oral use may include suspensions, internal liquid formulations, emulsions, or syrups, and various excipients, such as wetting agents, sweetening agents, air fresheners, or preservatives in addition to water or liquid paraffin, which are commonly used simple diluents.

In addition, in some cases, cross-linked polyvinylpyrrolidone, agar, alginic acid or sodium alginate may be added as a disintegrating agent, and an anti-aggregating agent, a lubricant, a wetting agent, a flavoring agent, an emulsifier, and a preservative may be additionally included.

When administered parenterally, the pharmaceutical composition of the present invention may be formulated according to methods known in the art in the form of injections, transdermal administration agents and nasal inhalants together with suitable parenteral carriers. In the case of the injection, it must be sterilized and protected from contamination of microorganisms such as bacteria and fungi. Examples of suitable carriers for injection may include, but are not limited to, water, ethanol, polyol (e.g., glycerol, propylene glycol and liquid polyethylene glycol, etc.), mixtures thereof and/or a solvent or dispersion medium containing vegetable oil. More preferably, suitable carriers may include Hanks' solution, Ringer solution, phosphate buffered saline (PBS) with triethanolamine or isotonic solutions such as sterile water for injection, 10% ethanol, 40% propylene glycol and 5% dextrose, etc. In order to protect the injection from microbial contamination, it may further include various antibacterial and antifungal agents such as parabens, chlorobutanol, phenol, sorbic acid, thimerosal, and the like. In addition, in most cases, the injection may further include an isotonic agent such as sugar or sodium chloride.

In the case of transdermal administration agent, forms such as ointment, cream, lotion, gel, external solution, pasta, liniment, and air roll are included. In the above, 'transdermal administration' means that an effective amount of the active ingredient contained in the pharmaceutical composition is delivered into the skin by topically administering the pharmaceutical composition to the skin.

In the case of inhalant, the compounds for use according to the present invention may be conveniently delivered in the form of an aerosol spray from pressurized packs or nebulizers using a suitable propellant, for example, dichlorofluoromethane, trichlorofluoromethane, dichlorotetrafluoroethane, carbon dioxide or other suitable gas. In the case of a pressurized aerosol, the dosage unit may be determined by providing a valve to deliver a metered amount. For example, gelatin capsules and cartridges for use in inhalers or insufflators may be formulated to contain a compound and a suitable powder base powder mixture such as lactose or starch. Formulations for parenteral administration are described in Remington's Pharmaceutical Science, 15th Edition, 1975. Mack Publishing Company, Easton, Pennsylvania 18042, Chapter 87: Blaug, Seymour, a formulary commonly known to all pharmaceutical chemistry.

When the pharmaceutical composition of the present invention contains Cyclo-HisPro in an effective amount, it is possible to provide a desirable effect of preventing, ameliorating or treating fibrosis. As used herein, the term "effective amount" refers to an amount that exhibits a response greater than that of a negative control group, and preferably refers to an amount sufficient to prevent, ameliorate or treat fibrosis. The pharmaceutical composition of the present invention may contain 0.01 to 99.9% of Cyclo-Hispro, and the remaining amount may be occupied by a pharmaceutically acceptable carrier. The effective amount of Cyclo-HisPro included in the pharmaceutical composition of the present invention will vary depending on the form in which the composition is commercialized.

The total effective amount of the pharmaceutical composition of the present invention may be administered to a patient as a single dose, and may be administered by a fractionated treatment protocol in which multiple doses are administered for a long period of time. The pharmaceutical composition of the present invention may vary the content of active ingredients according to the severity of the disease. For example, it may be administered in one to several divided doses to be administered in an amount of preferably 0.001 to 100 mg, more preferably 0.01 to 10 mg per 1 kg of body weight per day based on Cyclo-HisPro. However, in the case of the dose of Cyclo-HisPro, since the effective dosage for a patient is determined in consideration of various factors such as administration route of the pharmaceutical composition and number of treatments, as well as the patient's age, weight, health status, sex, disease severity, diet, and excretion rate, in consideration of this point, one of ordinary skill in the art will be able to determine an appropriate effective dosage of the Cyclo-HisPro according to a specific use for preventing, treating or ameliorating fibrosis. The pharmaceutical composition according to the present invention is not particularly limited in its formulation, administration route and administration method as long as the effect of the present invention is exhibited.

The pharmaceutical composition for preventing or treating fibrosis of the present invention may be used alone or in combination with methods using surgery, radiation therapy, hormone therapy, chemotherapy, or biological response modifiers.

The pharmaceutical composition for preventing or treating fibrosis of the present invention may also be provided in the formulation of an external application containing Cyclo-HisPro. In this aspect, the composition of the present invention may be a quasi-drug composition for preventing or ameliorating fibrosis and a quasi-drug comprising the composition.

The external application may be directly applied to the skin or oral cavity. When the pharmaceutical composition for preventing or treating fibrosis of the present invention is used for external application, it may further contain adjuvants commonly used in the field of dermatology, such as any other ingredients commonly used for external applications for skin including fatty substance, organic solvent, solubilizer, thickening and gelling agent, emollient, antioxidant, suspending agent, stabilizer, foaming agent, air freshener, surfactant, water, ionic emulsifier, nonionic emulsifier, filler, sequestering agent, chelating agent, preservative, vitamin, blocking agent, wetting agent, essential oil, dye, pigment, hydrophilic active agent, lipophilic active agent or lipid vesicle. In addition, the above ingredients may be introduced in an amount generally used in the field of dermatology.

When provided for external application, the composition of the present invention may be in the formulation of a liquid, ointment, patch, gel, cream or spray, but is not limited thereto. According to an embodiment of the present invention, the quasi-drugs of the present invention may include toothpaste, oral care products including mouthwash and mouth spray, ointments, masks, poultices, plasters, and transdermal absorbents.

When the composition of the present invention is used as a quasi-drug composition, Cyclo-HisPro may be added as it is or may be appropriately used in combination with other quasi-drug ingredients according to a conventional method. The mixing amount of the active ingredients may be appropriately determined according to the purpose of use (prevention, health or therapeutic treatment).

The contents of the pharmaceutical composition and health functional food composition of the present invention may be applied mutatis mutandis to the quasi-drug composition and quasi-drug of the present invention.

As used herein, the term "health functional food" includes both the meanings of "functional food" and "health food".

As used herein, the term "functional food" is the same term as food for special health use (FoSHU), and refers to food with high medical effects processed to efficiently exhibit bioregulatory functions in addition to nutritional supply.

As used herein, the term "health food" refers to food having an active health maintenance or promotion effect compared to general food, and health supplement food refers to food for the purpose of health supplementation. In some cases, the terms functional food, health food, and health supplement food are used interchangeably. The above food may be prepared in various forms such as tablets, capsules, powders, granules, liquids, pills, etc. in order to obtain a useful effect in improving and recovering liver function.

As a specific example of such a functional food, by using the composition, it is possible to produce a processed food with good storage properties while at the same time being transformed by taking advantage of the characteristics of agricultural products, livestock products or aquatic products.

The health functional food composition of the present invention may also be prepared in the form of nutritional supplements, food additives and feeds, and is intended for consumption by animals, including humans or livestock.

Food compositions of this type can be prepared in various forms according to conventional methods known in the art. It may be prepared as regular food by adding Cyclo-HisPro to the following food including, but not limited to, beverages (including alcoholic beverages), fruits and their processed foods (e.g., canned fruit, bottled, jam, marmalade, etc.), fish, meat and their processed foods (e.g., ham, sausages, corn beef, etc.), breads and noodles (e.g., udon noodles, soba noodles, ramen, spaghetti, macaroni, etc.), fruit juice, various drinks, cookies, yeot (Korean hard taffy), dairy products (e.g., butter, cheese, etc.), edible vegetable oils and fats, margarine, vegetable protein, retort food, frozen food, various seasonings (e.g., soybean paste, soy sauce, sauce, etc.).

In addition, it may be prepared as the nutritional supplement by adding Cyclo-HisPro to capsules, tablets, pills, and the like, but it is not limited thereto.

For health functional food, the Cyclo-HisPro may be prepared in the form of tea, juice, and drink and ingested by liquefaction, granulation, encapsulation, and powder so that it can be drinkable (as health drink), but it is not limited thereto. In addition, in order to use the Cyclo-HisPro in the form of a food additive, it may be prepared and used in the form of a powder or a concentrate. In addition, it may be prepared in the form of a composition by mixing the Cyclo-HisPro with an active ingredient known to be effective in preventing or ameliorating fibrosis.

When the food composition of the present invention is used as a health drink composition, the health drink composition may contain various flavoring agents or natural carbohydrates as additional ingredients, like a conventional drink. The above-mentioned natural carbohydrates may be monosaccharides such as glucose and fructose; disaccharides such as maltose and sucrose; polysaccharides such as dextrin and cyclodextrin; or sugar alcohols such as xylitol, sorbitol, and erythritol. As sweetening agents, natural sweetening agents such as taumatine and stevia extract, synthetic sweetening agents such as saccharin and aspartame, or the like may be used. The proportion of the natural carbohydrate is generally about 0.01 to 0.04 g, preferably about 0.02 to 0.03 g per 100 mL of the composition of the present invention.

Cyclo-HisPro may be contained as an active ingredient in a food composition for preventing or ameliorating fibrosis, and the amount may be an effective amount to obtain the preventing or ameliorating effect, for example, preferably 0.01 to 100% by weight based on the total weight of the total composition, but not particularly limited thereto. The food composition of the present invention may be prepared by mixing Cyclo-HisPro with other active ingredients known to be effective in preventing or ameliorating fibrosis.

In addition to the above, the health functional food of the present invention may include various nutritional supplements, vitamins, electrolytes, flavoring agents, coloring agents, pectic acid, pectic acid salts, alginic acid, alginic acid salts, organic acids, protective colloidal thickeners, pH regulators, stabilizers, preservatives, glycerin, alcohols, or carbonating agents, and the like. In addition, the health food of the present invention may contain fruit flesh for the production of natural fruit juice, fruit juice beverage, or vegetable beverage. These ingredients may be used independently or in combination. The proportion of these additives is not particularly important, but is generally selected in the range of 0.01 to 0.1 parts by weight per 100 parts by weight of the composition of the present invention.

The present invention also relates to a method for preventing or treating fibrosis comprising administering to a subject in need thereof an effective amount of Cyclo-HisPro.

In the method of the present invention, the term "subject" includes any animal (e.g., human, horse, pig, rabbit, dog, sheep, goat, non-human primate, cow, cat, guinea pig or rodent), but is not limited thereto. These terms do not denote a specific age or sex. Accordingly, whether female or male, adult and neonatal subjects as well as fetuses are intended to be included. A patient refers to a subject with a disease or disorder. The term patient includes human and veterinary subjects.

In the method of the present invention, the description of the constitution including the effect of CHP, its administration route, the number of administrations, the dosage, etc. is the same as described above, and thus description thereof will be omitted.

The present invention also provides a use of Cyclo-HisPro in the manufacture of a pharmaceutical composition for preventing or treating fibrosis.

Hereinafter, the present invention will be described in more detail through examples. However, since the present invention may have various changes and may have various forms, the specific examples and descriptions described below are only for helping the understanding of the present invention, and it is not intended to limit the invention to any particular form disclosed. It should be understood that the scope of the present invention includes all modifications, equivalents and substitutes included in the spirit and scope of the present invention.

### [Mode for Carrying Out the Invention]

### [Preparation example]

CHP (Cyclo-HisPro) used in the following examples was purchased from Bachem and used.

### [Example 1]

### Confirmation of antifibrotic effect in human-derived proximal tubular epithelial cells according to CHP treatment

### 1-1. Isolation and culture of human-derived proximal tubular epithelial cells

As shown in FIG. 1, human-derived glomerular endothelial cells and human-derived proximal tubular epithelial cells, which can be used for cell experiments, were purely isolated and cultured by isolating glomeruli and tubular interstitial tissue from normal adult kidney tissue and primarily culturing them.

### 1-2. Observation of cytological morphology after CHP treatment in a human-derived proximal tubular epithelial cell fibrosis model

By inducing cell fibrosis in human-derived proximal tubular epithelial cells that were isolated and primarily cultured in Example 1-1 by treating recombinant TGFβ (2 ng/ml) to the cells, cytoskeletal remodeling and morphological changes (became thin, elongated and structure disappeared) were occurred, which confirming that fibrosis was properly induced (FIG. 2).

Thereafter, it was confirmed cytomorphologically that fibrosis was improved when various concentrations of CHP (4, 20 and 100 µg/ml) were treated (FIG. 2).

### 1-3. Confirmation of expression of E-cadherin, fibronectin and pSTAT3 proteins after CHP treatment in a human-derived proximal tubular epithelial cell fibrosis model

Under the same conditions as in Example 1-2, the expression levels of proteins of E-cadherin, a junction marker, fibronectin, a fibrosis marker, and pSTAT3, a fibrosis transcription factor, after CHP administration were checked by Western blot.

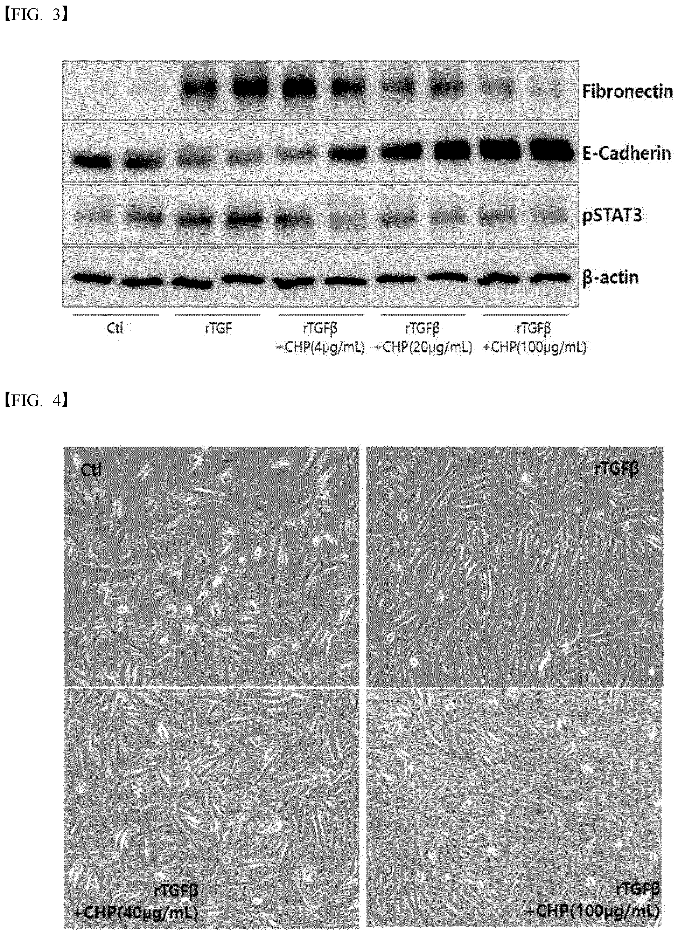

As a result, as shown in FIG. 3, it was confirmed that the protein expression of E-cadherin was restored and the protein expression of fibronectin and pSTAT3 decreased according to the CHP administration.

### [Example 2]

### Confirmation of antifibrotic effect in human-derived glomerular endothelial cells according to CHP

### Observation of cytological morphology after CHP treatment in a human-derived glomerular endothelial cell fibrosis model

As in the human-derived proximal tubular epithelial cell fibrosis model of Example 1-1, by inducing cell fibrosis in glomerular endothelial cells by treatment with rTGFβ (2 ng/ml), cytoskeletal remodeling and morphological changes were occurred, which confirming that fibrosis was properly induced (FIG. 4).

Thereafter, it was confirmed cytomorphologically that fibrosis was improved when various concentrations of CHP (40 and 100 µg/ml) were treated (FIG. 4).

Through the above results, it was possible to confirm the excellent therapeutic effect of CHP on renal fibrosis.

### [Example 3]

### Confirmation of antifibrotic effect in human-derived hepatocytes according to CHP treatment

### 3-1. CHP treatment after culturing human-derived hepatocytes and inducing fibrosis thereof

DMEM medium and FBS were purchased from Hyclone for culturing the Huh7 liver cell line, and TGFβ for inducing fibrosis was purchased from R&D Systems. The cells were cultured in a DMEM medium supplemented with 10% FBS in a 5% CO₂ incubator at 37 °C, treated with TGFβ at a concentration of 2 ng/ml and treated with CHP at 0, 62.5, 125, or 250 ng/ml, and cells were harvested at 48 hours of treatment.

### 3-2. Confirmation of fibronectin protein expression after CHP treatment in a human-derived hepatocyte fibrosis model

RIPA lysis buffer and Halt^{™} protease and phosphatase inhibitor cocktails was purchased from Thermo to analyze the expression of fibronectin protein, a fibrotic protein, and for protein quantification, BCA protein quantification kit was purchased from Thermo. For Western blot, Thermo's Bolt^{™} protein gel electrophoresis system and Bio-rad's wet transfer system were prepared. The antibody of fibronectin protein, a marker of liver fibrosis, and the GAPDH antibody used as a loading control were purchased from Abcam. 100 ul of RIPA buffer containing protease and phosphatase inhibitors was added to the harvested Huh7 cells, and the cells were lysed by pipetting. After leaving on ice for 10 minutes, centrifugation was performed at 15,000 rpm at 4 °C for 10 minutes. The supernatant was collected, and the protein concentration was measured by BCA quantitation, and the protein was separated from the same amount of samples using the Bolt^{™} protein gel electrophoresis system, and transferred to a nitrocellulose membrane. The membrane was blocked with 5% skim milk at room temperature for 1 hour, and then reacted with fibronectin and GAPDH antibodies, which are primary antibodies, overnight at 4 °C. After washing 3 times for 10 minutes with TBST, the reaction was performed with the secondary antibody at room temperature for 1 hour. After washing 3 times for 10 minutes with TBST, the expression level was measured by reacting with ECL. The size of the appearing band was quantified using the ImageJ program and corrected by dividing the fibronectin band size value by the GAPDH band size value. Statistical significance was analyzed using Student's t-test statistical method with the control group. #p<0.05 (negative control group: TGFβ treated control group).

As a result of the experiment, as shown in FIG. 5a-b, it was confirmed that the expression level of fibronectin protein, a fibrotic protein, which increases according to TGFβ treatment, showed a tendency to decrease when treated with CHP 250 ng/ml together with TGFβ. Through this, it was confirmed that CHP can ameliorate fibrosis in the liver cell line Huh7 cells.

### [Example 4]

### Confirmation of antifibrotic effect in an animal model of liver fibrosis according to CHP administration

Long-term high-fat diet intake is known to be a risk factor for liver fibrosis. An inflammatory environment is induced in the liver tissue due to insulin resistance and increased blood fatty acids, adipokines, and cytokines that appear when a fatty diet is consumed for a long period of time, and the interaction of immune cells accumulated in the liver tissue proceeds with fibrosis, in particular, IL-13 cytokine secreted by Th2 CD4+ T cells causes macrophages to produce large amounts of TGFβ, a key inducing factor for fibrosis. Signal transduction by TGFβ causes transformation of cells in liver tissue and promotes fibrosis progression (Rosselli, M et al., Current Pharmaceutical Design, 20(31), 5010-5024, 2014; Wu. D et al., Science, 332:243-7, 2011; J Investig Med, 60(8): 1147-1150, 2012; Fichtner-Feigl S et al., Nat Med, 12:99-106, 2006; Lee CG et al., J Exp Med, 194:809-21, 2001).

When normal mice were fed a high-fat diet containing 60% fat for 10 to 16 weeks, liver fibrosis was observed, which can be confirmed through the expression of fibronectin, a representative fibrosis marker protein (Kim, I. H et al., AGE, 38(4), 291-302, 2016; Chen, H. J., & Liu, J., Biomedicine & Pharmacotherapy, 97, 1386-1396, 2018.1-2).

### 4-1. Design of experimental animals and induction of liver fibrosis

For liver fibrosis experiments in animals, 12-week-old C57BL/6 mice fed a high-fat diet containing 60% fat for 6 weeks were purchased from Jackson, USA, and Research diets D12492, a 60% fat-containing high-fat dietary feed purchased from Saeron Bio, were ingested for 17 weeks. Mice were bred to allow free access to feed and water in an environment of 24±3 °C. C57BL/6 mice fed a high-fat diet for 6 weeks were acclimatized to the breeding environment for 1 week, and their weights were measured and evenly divided into 3 groups as shown in Table 1. CHP was orally administered to each group at a concentration of 5 mg/kg or 35 mg/kg once a day for 16 weeks, and the same amount of distilled water was orally administered to the control group in the same manner.

**[Table 1]**

| Group | HFD | CHP5 | CHP35 |
|---|---|---|---|
| Administra tion | Vehicle (water) | CHP 5 mg/kg | CHP 35 mg/kg |
| Number of mice | 10 | 8 | 8 |

### 4-2. Confirmation of the expression of fibronectin protein, a marker of fibrosis, in an animal model of liver fibrosis administered with CHP

Isoflurane required for anesthesia and dissection of mice was purchased from Hana Pharmaceutical, and Vetequip's RC2 Rodent Circuit Controller Anesthesia System was prepared. Phosphate buffered saline (PBS) was purchased from Hyclone. To isolate the mouse liver tissue, the mice were anesthetized by respiratory anesthesia with 3-3.5% isoflurane. After blood was drawn from the heart of anesthetized mice, the liver tissue was immediately excised and washed with PBS. Then, 50 mg of the liver tissue was cut out and placed in 500 ul of RIPA buffer containing protease and phosphatase inhibitors, and was pulverized using IKA's T10 homogenizer. After leaving on ice for 15 minutes, centrifugation was performed at 15,000 rpm at 4 °C. Western blotting was performed in the same manner as in Example 3-2 to analyze the expression of fibronectin protein, a fibrotic protein. Statistical significance was analyzed using the Student's t-test statistical method with HFD control group (^{∗}p<0.05, ^{∗∗}p<0.01).

As a result of the experiment, as shown in FIG. 6a-b, it was confirmed that the expression level of fibronectin protein, a representative fibrosis marker, was significantly decreased in the CHP-administered group, and the effect was confirmed to be stronger according to the concentration of CHP. This means that CHP ameliorates liver fibrosis, which is a clear evidence that it can be applied to the treatment of liver fibrosis.

### [Example 5]

### Confirmation of antifibrotic effect in human-derived lung cells according to CHP treatment

### 5-1. CHP treatment after culturing human-derived lung cells and inducing fibrosis thereof

MEM medium and FBS were purchased from Hyclone for culturing the L-132 lung cell line, and TGFβ for inducing fibrosis was purchased from R&D Systems. The cells were cultured in a MEM medium supplemented with 10% FBS in a 5% CO₂ incubator at 37 °C, treated with TGFβ at a concentration of 2 ng/ml and treated with CHP at 0, 62.5, or 125 ng/ml, and cells were harvested at 48 hours of treatment.

### 5-2. Confirmation of fibronectin protein expression after CHP treatment in a human-derived lung cell fibrosis model

Western blotting was performed in the same manner as in Example 3-2 to analyze the expression of fibronectin protein, a fibrotic protein. Statistical significance was analyzed using Student's t-test statistical method with the control (#p<0.05 (negative control group: TGFβ-treated control group), ^{∗∗}p<0.01 (TGFβ-treated control group: CHP 125 ng/ml-treated group)).

As a result of the experiment, as shown in FIG. 7a-7b, it was confirmed that the expression level of fibronectin protein, a fibrotic protein, which increases according to TGFβ treatment, was significantly decreased when treated with CHP 125 ng/ml together with TGFβ. Through this result that CHP ameliorated fibrosis in L-132 cells, a lung cell line, it was confirmed that CHP exhibited an excellent therapeutic effect on lung fibrosis.

### [Example 6]

### Confirmation of antifibrotic effect in an animal model of pulmonary fibrosis according to CHP administration

Pulmonary fibrosis is caused by prolonged high-fat diet intake. This appears to be due to an increase in the level of inflammation caused by high fat intake, and it has been reported that pulmonary fibrosis appeared when normal mice were fed a high-fat diet containing 60% fat for 15 weeks (Ge, X. N et al., Experimental Lung Research, 39(9), 365-378, 2016). Specifically, long-term intake of high-fat diet induces obesity, and adipokines and cytokines secreted by hypertrophic adipose tissue and immune cells gathered here disrupt the migration of immune cells from bone marrow to lungs and cause chronic inflammatory response (de Vries A et al., Clin Exp Allergy, 39:731-739, 2009). In addition, IL-13 cytokine secreted by Th2 CD4+ T cells causes large amounts of TGFβ, a key fibrosis-inducing factor, to be produced, and signal transduction by TGFβ promotes lung tissue transformation and fibrosis (Fichtner-Feigl S et al., Nat Med, 12:99-106, 2006; Lee CG et al., J Exp Med, 194:809-21, 2001). Pulmonary fibrosis can be confirmed through the expression level of the TGFβ gene increased compared to normal (Ge, X. N et al., Experimental Lung Research, 39(9), 365-378, 2016).

### 6-1. Design of experimental animals and induction of pulmonary fibrosis

For pulmonary fibrosis experiments in animals, an animal model of pulmonary fibrosis was established in the same manner as in Example 4-1.

### 6-2. Confirmation of the expression of fibrosis markers TGFβ and Collagen 3 genes in an animal model of pulmonary fibrosis administered with CHP

Isoflurane required for anesthesia and dissection of mice was purchased from Hana Pharmaceutical, and Vetequip's RC2 Rodent Circuit Controller Anesthesia System was prepared. Phosphate buffered saline (PBS) was purchased from Hyclone. To isolate the mouse lung tissue, the mice were anesthetized by respiratory anesthesia with 3-3.5% isoflurane. After blood was drawn from the heart of the anesthetized mice, the lung tissue was immediately excised, 50 mg of the lung was cut out, and 500 µL of NucleoZOL was added thereto, followed by pulverization using a T10 homogenizer from IKA. Then, RNA was extracted according to NucleoZOL's Total RNA Isolation protocol, and cDNA was synthesized from 1 µg of RNA by reverse transcription polymerase chain reaction using the iScript cDNA synthesis kit. The synthesized cDNA was analyzed by real-time PCR with iQ SYBR Green Supermix using forward/reverse primer sets corresponding to each gene. The expression value of each gene was corrected by dividing by the expression value of *GAPDH*, a housekeeping gene. Primers for real-time PCR were synthesized and used by Bioneer with the sequence shown in Table 2.

**[Table 2]**

| **Gene name** | **Forward primer** | **SEQ ID NO:** | **Reverse primer** | **SEQ ID NO:** |
|---|---|---|---|---|
| TGFβ | 5'-cctgagtggctgtcttttga-3' | 1 | 5'-aatcgaaagccctgtattccg-3' | 2 |
| *Collagen 3* | 5'-agtcaaggagaaagtggtcg-3' | 3 | 5'-ccagggaaacccatgacac-3' | 4 |
| *GAPDH* | 5'-acactgagcaagagagaggc-3' | 5 | 5'-tgggggtctgggatggaaat-3' | 6 |

Statistical significance was analyzed using one-way ANOVA statistical analysis, and the Dunnett post hoc test was used to compare the significance with the control group (^{∗}p<0.05, ^{∗∗∗}p<0.001).

As a result of the experiment, as shown in FIG. 8, it was confirmed that the expression of TGFβ and *Collagen 3* genes, which are major fibrosis markers shown in the lungs, decreased more significantly according to the CHP administration concentration. This means that CHP ameliorates pulmonary fibrosis, confirming an excellent therapeutic effect in the treatment of pulmonary fibrosis.

### [Example 7]

### Confirmation of antifibrotic effect in human-derived skin cells according to CHP treatment

### 7-1. CHP treatment after culturing human-derived skin cells and inducing fibrosis thereof

DMEM medium and FBS were purchased from Hyclone for culturing the HS68 skin fibroblast-derived cell line, and TGFβ for inducing fibrosis was purchased from R&D Systems. The cells were cultured in a DMEM medium supplemented with 10% FBS in a 5% CO₂ incubator at 37 °C, treated with TGFβ at a concentration of 2 ng/ml and treated with CHP at 0, 62.5, 125, 250, or 500 ng/ml, and cells were harvested at 48 hours of treatment.

### 7-2. Confirmation of fibronectin protein expression after CHP treatment in a human-derived skin cell fibrosis model

Western blotting was performed in the same manner as in Example 3-2 to analyze the expression of fibronectin protein, a fibrotic protein. Statistical significance was analyzed using Student's t-test statistical method with the control (#p<0.05 (negative control group: TGFβ-treated control group), ^{∗}p<0.05 (TGFβ-treated control group: CHP 500 ng/ml-treated group)).

As a result of the experiment, as shown in FIG. 9a-9b, it was confirmed that the expression level of fibronectin protein, a fibrotic protein, which increases according to TGFβ treatment, was significantly decreased when treated with CHP 500 ng/ml together with TGFβ. Through this result that CHP ameliorated fibrosis in HS68 cells, a skin cell line, it was confirmed that CHP can be applied to the treatment of skin fibrosis.

### [Example 8]

### Confirmation of antifibrotic effect in an animal model of cardiac fibrosis according to CHP administration

Risk factors for heart fibrosis are well known, including poor diet, obesity, and high cholesterol. When a high-fat diet is consumed for a long period of time, hypervolemia due to obesity occurs, overloading the heart, and causing deformation of the contractile and diastolic functions of the heart. In addition, symptoms such as hyperglycemia, insulin resistance, hyperlipidemia, and chronic inflammation are known to play a decisive role in cardiac fibrosis (Kaltman AJ, Goldring RM., Am J Med, 60:645-653, 1976; Xia Y et al., Histochem Cell Biol, 131:471-481, 2009; Ulasova E et al., J Mol Cell Cardiol., 50:147-156, 2011; Lo CS et al., J Cell Biochem., 103:1999-2009, 2008; Cavalera, M et al., Translational Research, 164(4), 323-335, 2014). In particular, it is known that the increase in cardiac mast cells and their degranulation due to obesity provide fibrosis-inducing factors such as TGFβ to the heart tissue and further promote cardiac fibrosis (Kong P, Christia P, Frangogiannis NG, Cell Mol Life Sci., 71:549-574, 2014).

In addition, there is a report that cardiac fibrosis occurs when normal mice are fed a high-fat diet for a long period of time. It has been reported that when normal mice are fed a high-fat diet containing 60% fat for 14 to 22 weeks, cardiac fibrosis is induced, and changes in the expression levels of TGFβ and *Collagen* genes, which are increased compared to normal, can be confirmed (Li, W et al., Nutrition & Metabolism, 14(1);68, 2017; Ternacle, J et al., European Heart Journal, 18(11), 1283-1291, 2017; Zhao, Y et al., Biochimica et Biophysica Acta (BBA) - Molecular Basis of Disease, 1863(8), 1991-2000, 2017; Xu, Z., & Kong, X. Q., Biomedicine & Pharmacotherapy, 89, 991-1004, 2017).

### 8-1. Design of experimental animals and induction of cardiac fibrosis

For cardiac fibrosis experiments in animals, an animal model of cardiac fibrosis was established in the same manner as in Example 4-1.

8-2. Confirmation of the expression of fibrosis markers TGFβ, *Fibronectin, Collagen 1, Collagen 2, Collagen 3* 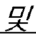 *Collagen* 4 genes in an animal model of cardiac fibrosis administered with CHP

Isoflurane required for anesthesia and dissection of mice was purchased from Hana Pharmaceutical, and Vetequip's RC2 Rodent Circuit Controller Anesthesia System was prepared. Phosphate buffered saline (PBS) was purchased from Hyclone. To isolate the mouse heart tissue, the mice were anesthetized by respiratory anesthesia with 3-3.5% isoflurane. After blood was drawn from the heart of the anesthetized mice, the heart tissue was immediately excised, 50 mg of the heart was cut out, and RNA extraction and gene expression analysis were performed in the same manner as in Example 6-2. Primers for real-time PCR were synthesized and used by Bioneer with the sequence shown in Table 3. The expression value of each gene was corrected by dividing by the expression value of β-actin, a housekeeping gene.

**[Table 3]**

| **Gene name** | **Forward primer** | **SE Q ID NO:** | **Reverse primer** | **SE Q ID NO:** |
|---|---|---|---|---|
| *Fibronectin* | 5'-ttggagaggagtgggagc-3' | 7 | 5'-gaaatgaccactgccaaagc-3 ' | 8 |
| TGFβ | 5'-cctgagtggctgtcttttga-3' | 1 | 5'-aatcgaaagccctgtattccg-3 ' | 2 |
| *Collagen 1* | 5'-ctaacgtggttcgtgaccg-3' | 9 | 5'-atccatcggtcatgctctct-3' | 10 |
| *Collagen 3* | 5'-agtcaaggagaaagtggtcg-3' | 3 | 5'-ccagggaaacccatgacac-3' | 4 |
| *Collagen 4* | 5'-cggtacacagtcagaccatt-3' | 11 | 5'-catcacgaaggaatagccga-3 ' | 12 |
| β-actin | 5'-gggaaggtgacagcattg-3 ' | 13 | 5'-atgaagtattaaggcggaagatt-3' | 14 |

Statistical significance was analyzed using one-way ANOVA statistical analysis, and the Dunnett post hoc test was used to compare the significance with the HFD control group (^{∗}p<0.05, ^{∗∗}p<0.01, ^{∗∗∗∗}p<0.0001).

As a result of the experiment, as shown in FIG. 10, it was confirmed that the expression of *Fibronectin,* TGFβ, *Collagen 1, 3, 4* genes, which are major fibrosis markers shown in the heart, was significantly reduced by CHP administration. However, a further decreased effect according to the concentration of CHP was not confirmed, which can be considered because a sufficient effect has already occurred with only a concentration of 5 mg/kg of CHP. These results that CHP inhibited the progression of cardiac fibrosis and ameliorated cardiac fibrosis suggest that CHP can be applied to the treatment of cardiac fibrosis.

### [Example 9]

### Confirmation of antifibrotic effect in an animal model of fat fibrosis according to CHP administration

It is known that the best way to prevent fibrosis is to block the inflammatory response. In particular, adipose tissue secretes inflammatory substances, causing a vicious cycle of fat fibrosis due to inflammation. It has been reported that fibrosis of adipose tissue occurs when normal mice are fed a high-fat diet containing 60% fat for 16 to 24 weeks (Hu, M et al., Evidence-Based Complementary and Alternative Medicine, 1-12, 2018; Kwon, E. Y, & Choi, M. S., Nutrients, 10(10), 1415, 2018; Nakazeki, F et al., Scientific Reports, 8(1), 2018; Muniappan, L et al. Scientific Reports, 7(1), 2017; Lancha, A et al., PLoS ONE, 9(5), e98398, 2014; Velαzquez, K. T et al., Physiological Reports, 5(18), e13412, 2017; Wang, L., Ye, X., Hua, Y, & Song, Y., Biomedicine & Pharmacotherapy, 105, 121-129, 2018). When a high-fat diet is consumed for a long period of time, adipocytes expand due to excessive nutrition, causing triglyceride accumulation, adipocyte death, production of adipokines and cytokines, endoplasmic reticulum stress, adipose tissue hypoxia, etc., resulting in infiltration of immune cells into adipose tissue and occurrence of chronic inflammation (Schenk S et al., J Clin Invest, 118:2992-3002, 2008; Sun K, Kusminski CM, Scherer PE, J Clin Invest, 121:2094-101, 2011). The interaction between various inflammatory immune cells accumulated in adipose tissue and macrophages-adipocytes produces TGFβ, a key inducing factor for fibrosis, which causes adipose tissue to be deformed, leading to fat fibrosis (Lee CG et al., J Exp Med, 194:809-21, 2001; Fichtner-Feigl S et al., Nat Med, 12:99-106, 2006; Pessin, J. E., & Kwon, H, Journal of Investigative Medicine, 60(8), 1147-1150, 2012). Fibrosis of adipose tissue can be confirmed through changes in the expression of representative fibrosis gene markers that are increased compared to normal adipose tissue.

### 9-1. Design of experimental animals and induction of fat fibrosis

For fat fibrosis experiments in animals, an animal model of fat fibrosis was established in the same manner as in Example 4-1.

### 9-2. Confirmation of the expression of fibrosis markers TGFβ, Fibronectin, Collagen 3 and CTGF genes in an animal model of fat fibrosis administered with CHP

Isoflurane required for anesthesia and dissection of mice was purchased from Hana Pharmaceutical, and Vetequip's RC2 Rodent Circuit Controller Anesthesia System was prepared. Phosphate buffered saline (PBS) was purchased from Hyclone. To isolate the mouse adipose tissue, the mice were anesthetized by respiratory anesthesia with 3-3.5% isoflurane. After blood was drawn from the heart of the anesthetized mice, the epididymal adipose tissue (EAT) was immediately excised, 100 mg of the fat was cut out, and RNA extraction and gene expression analysis were performed in the same manner as in Example 6-2. Primers for real-time PCR were synthesized and used by Bioneer with the sequence shown in Table 4. The expression value of each gene was corrected by dividing by the expression value of *GAPDH,* a housekeeping gene.

**[Table 4]**

| **Gene name** | **Forward primer** | **SE Q ID NO:** | **Reverse primer** | **SE Q ID NO:** |
|---|---|---|---|---|
| *Fibronectin* | 5'-ttggagaggagtgggagc-3' | 7 | 5'-gaaatgaccactgccaaagc-3 ' | 8 |
| TGFβ | 5'-cctgagtggctgtcttttga-3' | 1 | 5'-aatcgaaagccctgtattccg-3 ' | 2 |
| *Collagen 3* | 5'-agtcaaggagaaagtggtcg-3' | 3 | 5'-ccagggaaacccatgacac-3' | 4 |
| *CTGF* | 5'-ctgtgcctgccattacaact-3' | 15 | 5'-ccatgtctccgtacatcttcc-3' | 16 |
| *GAPDH* | 5'-acactgagcaagagagaggc-3' | 5 | 5'-tgggggtctgggatggaaat-3' | 6 |

Statistical significance was analyzed using one-way ANOVA statistical analysis, and the Dunnett post hoc test was used to compare the significance with the control group (^{∗}p<0.05, ^{∗∗}p<0.01).

As a result of the experiment, as shown in FIG. 11, it was confirmed that the expression of *Fibronectin,* TGFβ, *Collagen 3, CTGF* genes, which are major fibrosis markers shown in the fat, was markedly reduced by CHP administration. This means that CHP ameliorates fat fibrosis, confirming an excellent therapeutic effect in the treatment of fat fibrosis.

## Claims

1. A pharmaceutical composition for preventing or treating fibrosis comprising Cyclo-HisPro or a pharmaceutically acceptable salt thereof.

2. The pharmaceutical composition of claim 1, wherein the fibrosis develops in any one or more selected from the group consisting of: kidney, liver, lung, skin, heart, pancreas, urinary system, genital system, sweat gland, nerve, brain, bone marrow, muscle and joint.

3. The pharmaceutical composition of claim 1, wherein the fibrosis is any one or more selected from the group consisting of: pulmonary fibrosis, idiopathic pulmonary fibrosis, radiation-induced lung injury or lung fibrosis, pulmonary edema, cystic fibrosis, liver fibrosis, endomyocardial fibrosis, myocardial infarction, artrial fibrosis, glial scar, renal fibrosis, myelofibrosis, arthrofibrosis, fat fibrosis, skin fibrosis, neurofibrosis and myofibrosis.

4. A health functional food composition for preventing or ameliorating fibrosis comprising Cyclo-HisPro or a sitologically acceptable salt thereof.

5. The health functional food composition of claim 4, wherein the fibrosis develops in any one or more selected from the group consisting of: kidney, liver, lung, skin, heart, pancreas, urinary system, genital system, sweat gland, nerve, brain, bone marrow, muscle and joint.

6. The health functional food composition of claim 4, wherein the fibrosis is any one or more selected from the group consisting of: pulmonary fibrosis, idiopathic pulmonary fibrosis, radiation-induced lung injury or lung fibrosis, pulmonary edema, cystic fibrosis, liver fibrosis, endomyocardial fibrosis, myocardial infarction, artrial fibrosis, glial scar, renal fibrosis, myelofibrosis, arthrofibrosis, fat fibrosis, skin fibrosis, neurofibrosis and myofibrosis.

7. An antifibrotic composition comprising Cyclo-HisPro or a pharmaceutically acceptable salt thereof.

8. A method for preventing or treating fibrosis comprising administering to a subject in need thereof an effective amount of Cyclo-HisPro.

9. The method of claim 8, wherein the fibrosis develops in any one or more selected from the group consisting of: kidney, liver, lung, skin, heart, pancreas, urinary system, genital system, sweat gland, nerve, brain, bone marrow, muscle and joint.

10. The method of claim 8, wherein the fibrosis is any one or more selected from the group consisting of: pulmonary fibrosis, idiopathic pulmonary fibrosis, radiation-induced lung injury or lung fibrosis, pulmonary edema, cystic fibrosis, liver fibrosis, endomyocardial fibrosis, myocardial infarction, artrial fibrosis, glial scar, renal fibrosis, myelofibrosis, arthrofibrosis, fat fibrosis, skin fibrosis, neurofibrosis and myofibrosis.

11. A use of Cyclo-HisPro in the manufacture of a pharmaceutical composition for preventing or treating fibrosis.

12. The use of claim 11, wherein the fibrosis develops in any one or more selected from the group consisting of: kidney, liver, lung, skin, heart, pancreas, urinary system, genital system, sweat gland, nerve, brain, bone marrow, muscle and joint.

13. The use of claim 11, wherein the fibrosis is any one or more selected from the group consisting of: pulmonary fibrosis, idiopathic pulmonary fibrosis, radiation-induced lung injury or lung fibrosis, pulmonary edema, cystic fibrosis, liver fibrosis, endomyocardial fibrosis, myocardial infarction, artrial fibrosis, glial scar, renal fibrosis, myelofibrosis, arthrofibrosis, fat fibrosis, skin fibrosis, neurofibrosis and myofibrosis.
